(19) Europäisches Patentamt
European Patent Office
Office européen des brevets

(11) **EP 3 764 325 B1**

(12) **EUROPEAN PATENT SPECIFICATION**

(45) Date of publication and mention
of the grant of the patent:
**15.05.2024 Bulletin 2024/20**

(21) Application number: **20184932.0**

(22) Date of filing: **09.07.2020**

(51) International Patent Classification (IPC):
**G06T 11/00** $^{(2006.01)}$

(52) Cooperative Patent Classification (CPC):
**G06T 11/005; A61B 6/032; A61B 6/487;
A61B 6/5205; A61B 6/5258;** A61B 6/40;
A61B 6/482; A61B 6/504; G06T 2211/441

(54) **SYSTEM AND PROGRAM FOR AVOIDING FOCAL SPOT BLURRING**

SYSTEM UND PROGRAMM ZUR VERMEIDUNG VON BRENNFLECK-UNSCHÄRFE

SYSTÈME ET PROGRAMME POUR ÉVITER DU FLOU CAUSÉ PAR TACHE FOCALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.07.2019 US 201916510594
24.03.2020 JP 2020053357**

(43) Date of publication of application:
**13.01.2021 Bulletin 2021/02**

(73) Proprietor: **Canon Medical Systems Corporation
Otawara-shi, Tochigi 324-0036 (JP)**

(72) Inventors:
• **LEE, Tzu-Cheng
Tochigi, 324-0036 (JP)**
• **ZHOU, Jian
Tochigi, 324-0036 (JP)**
• **YU, Yu
Tochigi, 324-0036 (JP)**

(74) Representative: **Marks & Clerk LLP
15 Fetter Lane
London EC4A 1BW (GB)**

(56) References cited:
• JAN KUNTZ ET AL: "Focal spot deconvolution using convolutional neural networks", MEDICAL IMAGING 2019: PHYSICS OF MEDICAL IMAGING, March 2019 (2019-03), page 25, XP055746391, DOI: 10.1117/12.2513400 ISBN: 978-1-5106-2544-0

EP 3 764 325 B1

**Description**

BACKGROUND

In an X-ray system, when changing a focal spot size of an X-ray tube, there exists a trade-off between resolution and image quality.

For example, when a focal spot size of an X-ray becomes larger, the X-ray flux also becomes larger, which improves image quality, or SNR(Signal to Noise Ratio), of the projection image. However, when the focal spot size of the X-ray becomes larger, spatial resolution becomes lowered.

On the other hand, when the focal spot size of the X-ray becomes smaller, the resolution improves. However, the SNR of the projection image is lowered and the image quality is lowered.

JAN KUNTZ ET AL: "Focal spot deconvolution using convolutional neural networks", Proc. of SPIE Vol. 10948, March 2019, relates to the application of a convolutional neural network for de-blurring x-ray projection images.

SUMMARY OF INVENTION

In a first aspect, a system is provided in accordance with claim 1.

The first focal spot size may be equal to the third focal spot size.

The first focal spot size may be different from the third focal spot size.

The first trained model may be trained further using projection data being acquired using an X-ray source having a different focal spot size from the first focal spot size. The different focal spot size may be equal or similar to the third focal spot size.

The X-ray system may select a focal spot size from among the plurality of focal spot sizes according to a user instruction, scan protocol information, or examination region information.

When the X-ray system selects the third focal spot size, the circuitry may select the first trained model from among a plurality of trained models and apply acquired projection data with the selected focal spot size to the first trained model.

When the X-ray system selects a different focal spot size from the third focal spot size, the circuitry may select a different trained model from the first trained model from among a plurality of trained models and apply acquired projection data with the selected focal spot size to the different trained model.

The X-ray system may generate a second image by denoising the first image, said denoising being performed by inputting the first image into a second trained model.

The X-ray system may generate a second image by inputting the first image to a second trained model that generates, based on the first image, the second image having higher image quality than the first image.

The first trained model may be a trained model on which training is performed by using data acquired using only a detector array having detection elements of a first size as an input and using data acquired using only a detector array having detection elements of a second size as an output, the second size being smaller than the first size.

The first trained model may be a trained model on which training is performed by using data acquired using a detector array having detection elements of a first size as an input, and using data acquired using a detector array having detection elements of the first size and a detector array having detection elements of a second size as an output, the second size being smaller than the first size.

The first trained model may be a trained model on which training is performed by using data acquired using only a detector array having detection elements of a first size as an input, and using data acquired using only a detector array having detection elements of the first size and a detector array having detection elements of a second size as an output, the second size being smaller than the first size.

The first trained model is a trained model on which training is performed by using data acquired using a detector array having detection elements of a first size and a detector array having detection elements of a second size as an input, and using data acquired using a detector array having detection elements of the second size as an output, the second size being smaller than the first size.

The first trained model may be a trained model on which training is performed by using data acquired using only a detector array having detection elements of a first size and a detector array having detection elements of a second size as an input, and using data acquired using only a detector array having detection elements of the second size as an output, the second size being smaller than the first size.

The second size may be $1/n^2$ of the first size, wherein the n is an integer of 2 or more. The second size may be 1/4 of the first size.

The second size may be 1/9 of the first size.

The first projection data may be data acquired using a detector array having detection elements of a first size and the second projection data may be data acquired using a detector array having detection elements of a second size that is smaller than the first size.

The first projection data and the second projection data may be sinograms and the first image may be a computed tomography(CT) image.

The second projection data may be data acquired by averaging data detected by a plurality of detection elements of a detector array and a signal to noise ratio of the second projection data may be larger than a signal to noise ration of the first projection data.

The second projection data may be acquired by using a smaller point spread function than a point spread function of the first projection data, and a resolution of the fourth projection data is higher than a resolution of the third projection data.

The first trained model may be a residual network which is trained using first subtraction data between the first projection data and the second projection data and output second subtraction data by inputting the third projection data.

The fourth projection data may be generated based on the second subtraction data and the third projection data.

The fourth projection data may be generated by subtracting the second subtraction data from the third projection data.

The first projection data and the second projection data may be sinograms and the trained model may be trained such that weighting coefficients of a neural network is iteratively adjusted such that a loss function is minimized, the loss function being determined based on a disagreement between output sinogram and the second projection data, the output sinogram being output based on the first projection data.

The loss function may be at least one of a peak to noise ratio, a structural similarity index and an $l_p$-norm of a difference between the output sinogram and the second projection data.

The third projection data may be fluoroscopy data.

The third projection data may be X-ray CT projection data, and the X-ray system may further comprise a rotatable gantry, wherein the gantry comprises:

an X-ray tube configured on the gantry to rotate around an object, a size of a focal spot being controlled by an area of an electron beam on an anode and an angle of the anode with respect to a direction of an X-ray beam comprising X-rays emitted from the anode; and

a detector array including the plurality of detection elements, the detector array being arranged on the gantry diametrically opposed to the X-ray tube and configured to rotate together with the X-ray tube, the detector array being further configured to receive the X-rays to generate the X-ray CT projection data.

In a second aspect, a program is provided as recited in claim 11.

BRIEF DESCRIPTION OF THE DRAWINGS

Figure 1A is an image generated using a small focal spot and a relatively small X-ray exposure, according to one implementation;

Figure 1B is an image generated using a focal spot that is larger than the focal spot used to obtain Figure 1A and the exposure is twice as large as in Figure 1A, according to one implementation;

Figure 2 is an image showing a diagram of blurring in an X-ray projection image resulting from a finite width of a focal spot in the X-ray source, according to one implementation;

Figure 3A is an image illustrating tradeoffs for a small electron beam area combined with a large anode angle, according to one implementation;

Figure 3B is an image illustrating tradeoffs for a large electron beam area combined with a large anode angle, according to one implementation;

Figure 3C is an image illustrating shows tradeoffs for a large electron beam area combined with a small anode angle, according to one implementation;

Figure 4 is a diagram illustrating processing executed by an X-ray system according to an embodiment.

Figure 5 is a flowchart illustrating processing of training executed by an X-ray system according to an embodiment.

Figure 6 is a diagram illustrating an example of a DL network according to an embodiment.

Figure 7 is a diagram of an illustration of a case in which a convolutional neural network(CNN) is used as the DL network according to an embodiment.

Figure 8 is a diagram illustrating an example of an X-ray CT scanner according to an embodiment.

DETAILED DESCRIPTION

In the below, with references to the drawings, embodiments of an X-ray system, an image processing apparatus, and a program are explained in detail.

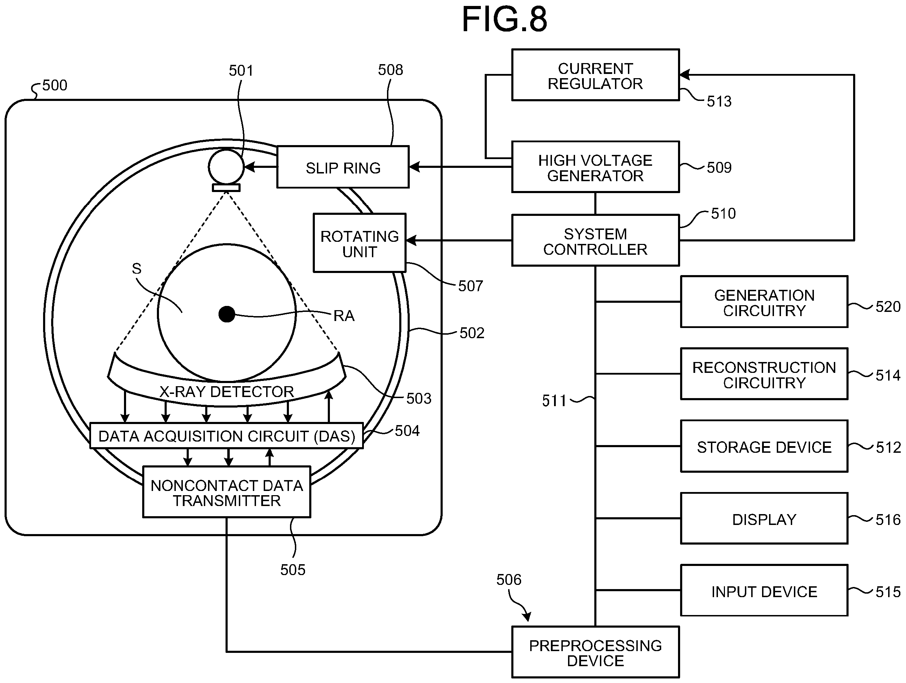

First of all, with reference to Figure 8, configuration of an X-ray system according to an embodiment is explained.

Figure 8 illustrates a non-limiting example of a CT scanner(an X-ray system). As shown in Figure 8, a radiography gantry 500 is illustrated from a side view and further includes an X-ray tube 501, an annular frame 502, and a multi-row or two-dimensional-array-type X-ray detector 503. The X-ray tube 501 and X-ray detector 503 are diametrically mounted across an object S on the annular frame 502, which is rotatably supported around a rotation axis RA.

The multi-slice X-ray CT apparatus further includes a high voltage generator 509 that generates a tube voltage applied to the X-ray tube 501 through a slip ring 508 so that the X-ray tube 501 generates X-rays. The X-rays are emitted towards the object S, whose cross sectional area is represented by a circle.

For example, the X-ray tube 501 having an average X-ray energy during a first scan that is less than an average X-ray energy during a second scan. Thus, two or more scans can be obtained corresponding to different X-ray energies. The X-ray detector 503 is located at an opposite side from the X-ray tube 501 across the object S for detecting the emitted X-rays that have transmitted through the object S. The X-ray detector 503 further includes individual detector elements or units.

The X-ray CT apparatus further includes other devices for processing the detected signals from X-ray detector 503. A data acquisition circuit or a Data Acquisition System (DAS) 504 converts a signal output from the X-ray detector 503 for each channel into a voltage signal, amplifies the signal, and further converts the signal into a digital signal.

The above-described data is sent to a preprocessing circuitry 506, which is housed in a console outside the radiography gantry 500 through a non-contact data transmitter 505. The preprocessing circuitry 506 performs certain corrections, such as sensitivity correction on the raw data. A storage 512 stores the resultant data, which is also called projection data at a stage immediately before reconstruction processing.

The storage 512 is connected to a system controller 510 through a data/control bus 511, together with a generation circuitry 520, reconstruction circuitry 514, input device 515, and display 516. The system controller 510 controls a current regulator 513 that limits the current to a level sufficient for driving the CT system.

The detectors are rotated and/or fixed with respect to the patient among various generations of the CT scanner systems. In one implementation, the X-ray tube 501 and the X-ray detector 503 are diametrically mounted on the annular frame 502 and are rotated around the object S as the annular frame 502 is rotated about the rotation axis RA.

The storage 512 can store the measurement value representative of the irradiance of the X-rays at the X-ray detector unit 503. Further, the storage 512 can store a dedicated program for executing method 10.

The reconstruction circuitry 514 can execute various steps of method 10. Further, reconstruction circuitry 514 can execute pre-reconstruction processing image processing such as volume rendering processing and image difference processing as needed. The reconstruction circuitry 514 is an example of the reconstruction unit.

The generation circuitry 520 performs various processes such as generating projection data, by inputting projection data to the trained model as described later. The processes executed by the generation circuitry 520 will be described later. The generation circuitry 520 is an example of a generation unit.

The pre-reconstruction processing of the projection data performed by the preprocessing circuitry 506 can include correcting for detector calibrations, detector nonlinearities, and polar effects, for example.

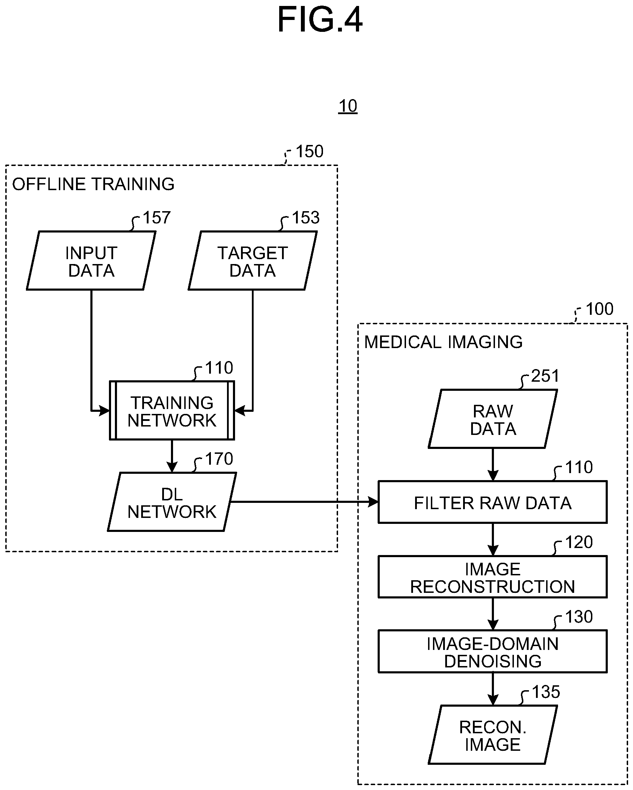

Post-reconstruction processing performed by the reconstruction circuitry 514 can include filtering and smoothing the image, volume rendering processing, and image difference processing as needed. The image reconstruction process can implement various steps of method 10 as illustrated in FIG. 4. The reconstruction circuitry 514 can use the memory to store, e.g., projection data, reconstructed images, calibration data and parameters, and computer programs.

The various circuitry (e.g., the reconstruction circuitry 514, generation circuitry 520 and preprocessing circuitry 506) can include a CPU (processing circuitry) that can be implemented as discrete logic gates, as an Application Specific Integrated Circuit (ASIC), a Field Programmable Gate Array (FPGA) or other Complex Programmable Logic Device (CPLD) .

An FPGA or CPLD implementation may be coded in VHDL, Verilog, or any other hardware description language and the code may be stored in an electronic memory directly within the FPGA or CPLD, or as a separate electronic memory.

Further, the storage 512 can be non-volatile, such as ROM, EPROM, EEPROM or FLASH memory. The storage 512 can also be volatile, such as static or dynamic RAM, and a processor, such as a microcontroller or microprocessor, can be provided to manage the electronic memory as well as the interaction between the FPGA or CPLD and the memory.

In one implementation, the reconstructed images can be displayed on a display 516. The display 516 can be an LCD display, CRT display, plasma display, OLED, LED or any other display known in the art.

Next, background regarding the embodiments will be described.

Practical constraints limit the smallest focal-spot size for X-ray tubes used as X-ray sources for projective imaging, and this limit to the focal-spot size in turn limits the resolution achievable in such applications as radiography, computed tomography, fluoroscopy, and angiography. These constraints include practical size limitations, heat transfer and material characteristics, dose constraints (e.g., maintaining the radiation dosage as low as reasonably possible), and time constraints. As a practical matter, a larger focal-spot size can generate a greater flux of X-rays resulting in a higher signal-to-noise ratio (SNR) in the projection images, but the larger focal-spot size comes at the cost a poorer spatial resolution.

The methods and apparatus described herein combine the best of large and small focal-spot sizes by acquiring a training dataset including projection images using both a large and a small focal-spot sizes, and then training a neural network to achieve the image resolution of a small focal-spot size from projection images acquired using a large focal-spot size without sacrificing the high SNR achieved with the large focal-spot size.

Thus, the methods described herein can generate projection images that have both good spatial resolution similar to an image generated using a small focal-spot size and the larger SNR of an image generated using a large focal-spot size. The importance of achieving good spatial resolution is becoming more significant as X-ray detector sizes continue to decrease.

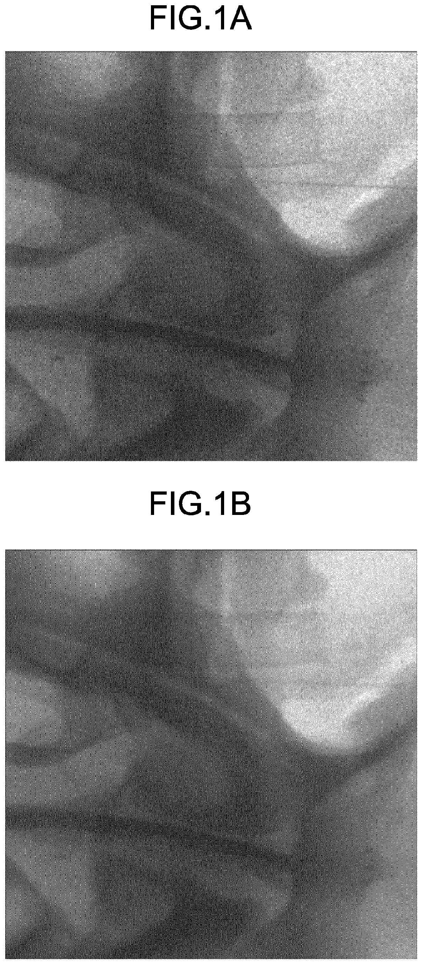

Referring now to the drawings, wherein like reference numerals designate identical or corresponding parts throughout the several views, Figures 1A and 1B show two projection images taken using different focal-spot sizes.

Figure 1A shows an X-ray projection image acquired using a smaller focal-spot size than the focal-spot size used to acquire the X-ray projection image shown in Figure 1B. The focal spot in Figure 1A is smaller than in Figure 1B, and the X-ray exposure from Figure 1B is twice as larger as in Figure 1A. Higher resolution is visible in Figure 1A than in Figure 1B, but Figure 1A sacrifices SNR in order to obtain this improved resolution. This is because the X-ray flux achievable using the smaller focal-spot size is less, resulting in smaller signals and thus lower SNRs in Figure 1A compared to Figure 1B.

Thus, it can be observed that focal-spot sizes, such as those used for the existing clinical systems, which are significantly large relative to the critical detector dimension adversely impact the overall system resolution. While the focal-spot size depends on particular design choices and trade-offs for a given X-ray imager, generally, the focal spot for all X-ray imagers is on the order of one millimeter due to the tube loading capacity for smaller focal spots.

Ideally, the focal-spot size would be made arbitrarily small, but that cannot be achieved due to X-ray tube design limitations. Ultimately, there is a trade-off between resolution and image quality. On the one hand, a larger focal-spot size can provide more exposure and greater SNR, but this greater SNR comes at the expense of poorer spatial resolution. On the other hand, smaller focal-spot sizes improve spatial resolution, but this improvement comes at the expense of less exposure and a smaller SNR.

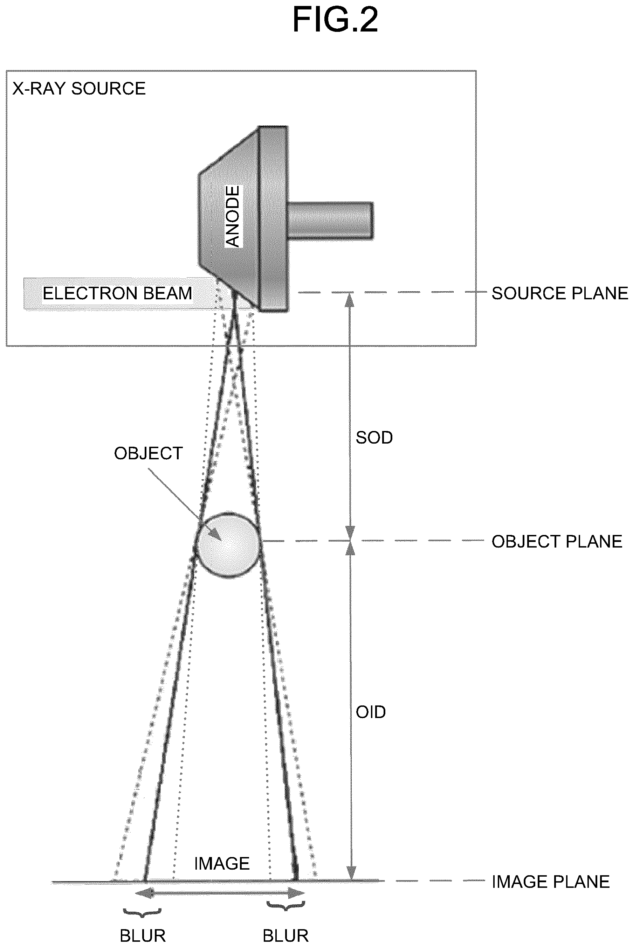

Fig. 2 illustrates an exemplary imaging system in which an object is imaged by X-rays from an X-ray source passing through the object being detected at an image plane. The size of the electron beam on the anode of the X-ray source determines the focal-spot size. The solid lines show the ray trajectories from a center of the focal spot and passing through the periphery of the object.

The dashed lines show the X-ray trajectories for X-rays from the edges of the focal spot passing through the periphery of the object. When the source-to-object distance (SOD) is much greater than the object-to-imaged distance (OID), the magnification is small and the point-spread function in the image plane is reduced. The magnification and the point-spread function of the image at the image plane can also be affected by the use an X-ray filter/lens (e.g., a butterfly filter) at the source. The relationship between the geometry of the X-ray apparatus and the focal-spot size to the image resolution and point-spread function are generally well understood and can be modeled using straightforward ray tracing, and, therefore, these details are not discussed herein.

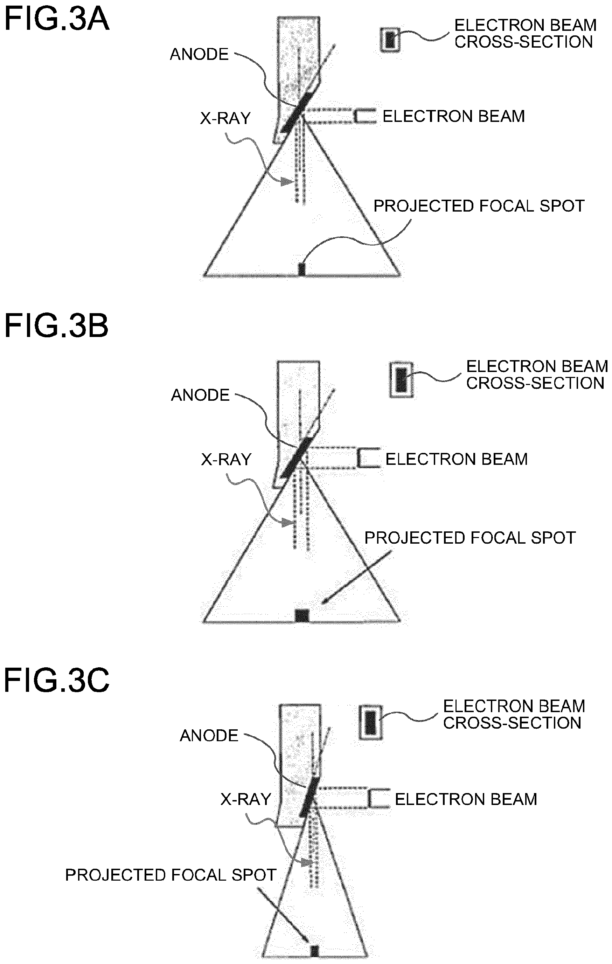

Figures 3A, 3B and 3C illustrate the tradeoffs between making the electron beam and/or the anode angle bigger or smaller.

In Figure 3A, the anode angle is large and the area of the electron beam is small, resulting in good field coverage (i.e., the field of view is large due to the large beam angle), small focal spot size for good resolution, but poor power loading (i.e., the X-ray flux is low resulting in either low SNR or long exposure time at a given view angle to compensate for the low flux rate).

In Figure 3B, the anode angle is large and the area of the electron beam is large, resulting in good field coverage, large focal spot size for poorer resolution, and good power loading. In Figure 3C, the anode angle is small and the area of the electron beam is large, resulting in narrower/poor field coverage, small focal spot size for good resolution, and good power loading. These tradeoffs also impact other aspects of CT imaging, such as manufacturing costs and limitations of the size of patients.

For examples, using a smaller effective focal spot size, results in lower power loading or smaller filed coverage, and, in turn, these factors limit the practicability of applying the finer focal size (e.g., 0.4x0.5 mm) to larger and more strongly attenuating patients (e.g., larger patients can require a larger beam angles and a higher mA setting, such as 500 mA).

Further, large power loading smaller effective focal spot size can increase the manufacturing costs.

Figure 4 shows a flow diagram for a non-limiting example of a method 10 that trains and uses a DL neural network 170 to perform data-domain corrections to X-ray projection data (e.g., edge/resolution enhancement, sinogram restoration, denoising, and/or artifact correction). Method 10, as illustrated in Figure 4, uses the DL network 170 to learn how to optimal filter raw data 105 (e.g., a sinogram), and then reconstructs a CT image from the Filtered data. Method 10 includes two parts: (i) an offline training process 150 and (ii) a medical imaging process 100.

That is, process 150 trains the DL network 170, and process 100 uses the trained DL network 170 to filter the raw data 251 in the projection domain, thereby generating high-quality images 135 with reduced noise and artifacts. In certain implementations such as fluoroscopy, steps 120 and 130 can be omitted and the output can be the corrected projection data.

In certain implementations, the DL network 170 includes a convolutional neural network (CNN) in which series of convolution, batch normalization, and rectified linear unit network layers are performed.

The network 170 is trained using process 160. In process 160, a loss function is used to iteratively adjust/optimize parameters of the DL network 170 (e.g., the parameters of the DL network 170 can include weighting coefficients connecting network layers, and activation functions/potentials of nodes within the layers). The optimization of the network parameters continues until stopping criteria are satisfied (e.g., a stopping criterion can be whether the value of the loss function converged to a predefined threshold) to generate the trained DL network 170.

The loss function compares target data 153 to an output generated by applying the input data 157 to a current version of the DL network 170. For example, the input data can include projection data acquired using a large focal spot size, and the target data can include projection data acquired using a small focal spot size and a large amount of averaging to reduce noise.

In other words, training of the DL network that is a first trained model is performed by using the input data 157 as an input and the target data 153 as an output, the input data 157 being a first projection data acquired by using an X-ray source of a first focal spot size, the target data 153 begin a second projection data acquired by using an X-ray source of a second focal spot size that is smaller than the first focal spot size.

Here, the second projection data is, for example, data being acquired by averaging data detected by a plurality of detectors. A signal to noise ratio of the second projection data is, for example, larger than a signal to noise ratio of the first projection data.

Alternatively, the second projection data may be acquired by using a smaller point spread function than a smaller point spread function of the first projection data.

Furhter, the input data 157 that is the first projection data and the target data 153 that is the second projection data, are, for example, sonograms.

In such a case, for a given CT scan, each low-quality (e.g., large focal spot size) sinogram of the input data forms a pair with the corresponding high-quality (e.g., small focal spot size) sinogram. The scans to acquire the low-quality sinograms for the input data 157 and the high-quality sinograms for the target data 153 can be performed on a phantom, for example.

Applying a low-quality sinogram from the input data to the current version of the DL network 170 generates an output from the network that is supposed to be a resolution-enhanced version of the low-quality sinogram (i.e., a filtered sinogram).

The DL network 170 is trained by iteratively adjusting the network coefficients in the DL network 170 to minimize the difference between the Filtered sinogram output from the DL network 170 and the high-quality sinogram from the target data 153.

The training of the network 170 is determined to be complete when the difference is minimized between the target data and the output from the DL network 170. The question of whether or not this difference has been sufficiently minimized is resolved based on one or more predetermined stopping criteria of process 160. Once the stopping criteria have been satisfied, the trained network 170 can then be stored and then later recalled to be used in the medical imaging process 100.

In alternative implementations, the DL network 170 is implemented as a residual network (ResNet). In other words, training of the DL network that is a first trained network may be performed by using a residual network.

In this case, the method described herein can filter an image by treating the difference between the small and large spot size sinograms as an additive residue that can be directly removed from the low-quality sinogram. This additive residue or difference image can be thought of as a high-pass filtered version of the small-spot-size projection data.

Thus, when a low-quality sinogram is applied to the neural network, the network outputs an image corresponding to the difference image. Then the corrected sinogram can be generated by subtracting the network output (the noise/artifact) from the low-quality sinogram to generate the corrected sinogram.

In method 10, a loss function is used to iteratively adjust network coefficients (e.g., weights and biases of convolutional and pooling layers) of the DL network 170 until stopping criteria are satisfied (e.g., convergence of the parameters to a predefined threshold) to generate the trained DL network 170. The loss function compares target data 153 that is high-quality data to results of a current version of the DL network 170 to which input data 157 is applied.

In other words, training of the DL network 170 that is a first trained model is performed such that weighting coefficients of the DL network 170 is iteratively adjusted to minimize the lose function determined based on the disagreement between the output sinogram and the target data 153, the output sinogram being output based on the input data 157 that is first projection data, the target data being the second projection data.

Process 100 is performed by obtaining raw data 251, e.g., by performing a CT scan to generate CT projections at a series of view angles (i.e., a low-quality sinogram). For example, the sinogram can be performed using a low-dose CT scan to generate the raw data 251.

Here, the raw data 251 is a third projection data acquired by using an X-ray source of a third focal spot size that is larger than the second focal spot size, the second focal spot size being a focal spot size of the target data 153 that is the second projection data acquired using an X-ray source of the second focal spot size.

In step 110 of process 100, the raw data 251 is filtered by applying the raw data 251 to the trained DL network 170. The DL network 170 then outputs a Filtered sinogram. In other words, the generation circuitry 520 generates a fourth projection data by inputting raw data 251 to the DL network 170 that is the first trained model, the raw data 251 being the third projection data acquired by using the X-ray source of the third focal spot size that is larger than the second focal spot size.

For example, when training of the DL network that is the first trained model is performed using a residual network, the generation circuitry 520 generates the fourth projection data by subtracting the output of the residual network from the third projection data.

It is noted that, in general, resolution of the fourth projection data is, for example, higher than the resolution of the third projection data.

It is noted that the third projection data may be, for example, fluoroscopy data.

In certain implementations, the DL network 170 is a convolution neural network (CNN). The CNN can be a network that directly generates local small sized filters, e.g.,

$$y_i = \sum_{j \in \text{Neighbor of } i} w_{ij} x_j$$

wherein $w_{ij}$ is the filter on the ith pixel.

In the training process 160, the same process as used in step 251 to generate the filtered sinograms from the raw data 105 is also used to generate output sinograms from the input data, and then compare, using the loss function, the output sinograms to the target data.

In step 120 of process 100, a CT image is reconstructed from the denoised sinogram. In other words, the reconstruction circuitry 514 reconstructs a fist image that is, for example, a CT image, based on the fourth projection data. Various methods can be used to reconstruct CT images from projection data, including filtered back-projection (FBP) and statistical iterative reconstruction (IR) algorithms.

In addition to FBP, other analytical methods can be used such as the Feldkamp Davis Kress (FDK) method Adaptive Iterative Dose Reduction 3D (AIDR 3D) method. Compared to FBP reconstruction methods, IR methods can provide improved image quality at reduced radiation doses.

One IR method performs unconstrained (or constrained) optimization to find the argument $p$ that minimizes the expression

$$\arg\min_{p} \left\{ \left\| \mathbf{A}\, p - \ell \right\|_W^2 + \beta U(p) \right\},$$

wherein $\ell$ is the projection data representing the logarithm of the X-ray intensity of projection images taken at a series of projection angles and $p$ is a reconstructed image of the X-ray attenuation for voxels/volume pixels (or two-dimensional pixels in a two-dimensional reconstructed image) in an image space. For the system matrix $\mathbf{A}$, each matrix value $a_{ij}$ ($i$ being a row index and $j$ being a column index) represents an overlap between the volume corresponding to voxel $p_j$ and the X-ray trajectories corresponding to projection value $\ell_i$. The data-fidelity term $\|Ap - \ell\|_W^2$ is minimized when the forward projection $\mathbf{A}$ of the reconstructed image $p$ provides a good approximation to all measured projection images $\ell$. Thus, the data fidelity term is directed to solving the system matrix equation $\mathbf{A}p = \ell$, which expresses the Radon transform (i.e., projections) of various rays from a source through an object S in the space represented by $p$ to X-ray detectors generating the values of $\ell$ (e.g., X-ray projections through the three-dimensional object *OBJ* onto a two-

dimensional projection image $\ell$).

The notation $\|g\|_W^2$ signifies a weighted inner product of the form $g^T W g$, wherein $W$ is the weight matrix (e.g., expressing a reliability of trustworthiness of the projection data based on a pixel-by-pixel signal-to-noise ratio). In other implementations, the weight matrix $W$ can be replaced by an identity matrix. When the weight matrix $W$ is used in the data fidelity term, the above IR method is referred to as a penalized weighted least squares (PLWS) approach.

The function $U(p)$ is a regularization term, and this term is directed at imposing one or more constraints (e.g., a total variation (TV) minimization constraint) which often have the effect of smoothing or denoising the reconstructed image. The value $\beta$ is a regularization parameter is a value that weights the relative contributions of the data fidelity term and the regularization term.

In step 130 of process 100, additional image-domain denoising is performed. This step is optional, and can be omitted in some implementations.

In other words, at Step 120, the generation circuitry 520 may generate a reconstructed image 135 that is a second image regarding which image quality is improved, based on the first image reconstructed at Step 120.

Example denoising methods include linear smoothing filters, anisotropic diffusion, non-local means, or nonlinear filters. Linear smoothing filters remove noise by convolving the original image with a convolution kernel that represents a low-pass filter or smoothing operation. For example, a Gaussian convolution kernel comprises elements determined by a Gaussian function. This convolution brings the value of each pixel into closer agreement with the values of its neighbors. Anisotropic diffusion removes noise while preserving sharp edges by evolving an image under a smoothing partial differential equation similar to the heat equation. A median filter is an example of a nonlinear filter and, if properly designed, a nonlinear filter can also preserve edges and avoid blurring. The median filter is one example of a rank-conditioned rank-selection (RCRS) filter, which can be applied to remove salt and pepper noise from an image without introducing significant blurring artifacts.

Additionally, a filter using a total-variation (TV) minimization regularization term can be applied if imaged region supports an assumption of uniformity over large areas that are demarked by sharp boundaries between the uniform areas. A TV filter is another example of a nonlinear filter. Moreover, non-local means filtering is an exemplary method of determining denoised pixels using a weighted average over similar patches within the images.

It is noted that the embodiments are not limited to this example. At Step 130, the generation circuitry 520 may perform denoise processing, by generating a reconstructed image 135 regarding which image quality is improved, from the first image, using a neural network different from the DL network. In other words, at step 130, the generation circuitry 520 generates the reconstructed image 135 that is the second image, based on the first image, by inputting the first image to a second trained model that generates the second image having higher image quality than the first image.

It is noted that the training of the second trained model is performed prior to the execution of the second trained model, by using, for example, image having low image quality as inputs and images having higher image quality than the input images.

Returning to Figure 4, finally, a reconstructed image 135 is output having good image quality, and the reconstructed image 135 can be displayed to a user or stored for later use.

Now a more detailed description of training a DL network is provided (e.g., training process 160). Here, the target data 153 are high-quality sinograms acquired using a small focal spot size in the X-ray tube, and the input data 157 are low-quality sinograms acquired using a large focal spot size, as described above.

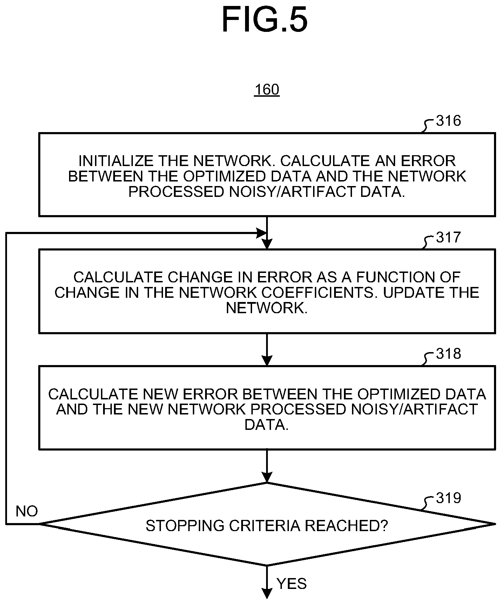

Figure 5 shows a flow diagram of one implementation of the training process 160. In process 160, input data 157 and target data 153 are used as training data to train a DL network 170, resulting in the DL network 170 being output from step 319 of process 160. The offline DL training process 160 trains the DL network 170 using a large number of input sonograms (input data 157) that are paired with corresponding target data 153 that is target sinograms 153 to train the DL network 170 to produce, from the input sinograms 157 that is input data, filtered sinograms resembling the target data 153 that is sinograms 153.

In training process 160, a set of training data is obtained, and the DL network 170 is iteratively updated to reduce the error (e.g., the value produced by a loss function). The DL network infers the mapping implied by the training data, and the cost function produces an error value related to the mismatch between the target data 153 (target sinograms) and the result produced by applying a current incarnation of the DL network 170 to the input sinograms (input data 157).

For example, in certain implementations, the cost function can use the mean-squared error to minimize the average squared error. In the case of a of multilayer perceptrons (MLP) neural network, the backpropagation algorithm can be used for training the network by minimizing the mean-squared-error-based cost function using a (stochastic) gradient descent method.

In step 316 of training process 160, an initial guess is generated for the coefficients of the DL network 170. For example, the initial guess can be based on *a priori* knowledge of the region being imaged or one or more exemplary denoising methods, edge-detection methods, and/or blob detection methods. Additionally, the initial guess can be based on one of a LeCun initialization, an Xavier initialization, and a Kaiming initialization.

Steps 316 through 319 of training process 160 provide a non-limiting example of an optimization method for training the DL network 170.

An error is calculated (e.g., using a loss function or a cost function) to represent a measure of the difference (e.g., a distance measure) between the target data 153( target sonograms) (i.e., ground truth) and the input data 157( input sonograms) after applying a current version of the network 170. The error can be calculated using any known cost function or distance measure between the image data, including those cost functions described above. Further, in certain implementations the error/loss function can be calculated using one or more of a hinge loss and a cross-entropy loss. In certain implementations, the loss function can be the $\ell_p$-norm of the difference between the target data and the result of applying the input data to the DL network 170. Different values of "p" in the $\ell_p$-norm can be used to emphasize different aspects of the noise. Further, a weighting mask (e.g., based on the attenuation coefficient of signal intensity) can be applied on a pixel-by-pixel basis to the difference between the target data and the result generated from the input data. In certain implementations, rather than minimizing an $\ell_p$-norm of the difference between the target data and the result from the input data, the loss function can represent a similarity (e.g., using a peak signal-to-noise ratio (PSNR) or a structural similarity (SSIM) index).

In other words, the loss function may be at least one of a peak to noise ratio, a structural similarity index and an $l_p$-norm of a difference between the output sinogram and the second projection data.

In certain implementations, the training is performed by minimizing the following loss function

$$\hat{\theta} = \arg \min_{\theta} \frac{1}{N} \sum_{n} \mathrm{L}\big(y'^{(n)}, f(y^{(n)}|\theta, \mathrm{h})\big) + \beta R(\mathrm{h})$$

where $\theta$ are the adjustable weighting coefficients of the DL network 170, h are the non-adjustable parameters (e.g., parameters selected by a user, such as the choice of reconstruction kernel), $y^{(n)}$ represents the $n$th input sinogram, $y'^{(n)}$ represents the $n$th target sinogram. The number $N$ is the total number of training data. In certain implementations, the following weighted mean absolute error loss function is used

$$\mathrm{L}(y', y) = \sum_{j} d_j |y_j - y'_j|$$

wherein $d_j$ is the weight which has the form

$$d_j = y'^{p}_{j}$$

with $p$ being a scalar. The choice of this weight is inspired by the statistical mean estimation method where $d_j$ is often necessarily chosen to be the inverse of data noise variance. To handle the overfitting issue an additional regularization R on h is used, which is given by $R(h) = \Sigma_j h_j$. The regularization strength can be tuned thru the parameter $\beta$.

In certain implementations, the DL network 170 is trained using backpropagation. Backpropagation can be used for training neural networks and is used in conjunction with gradient descent optimization methods. During a forward pass, the algorithm computes the network's predictions based on the current parameters $\theta$. These predictions are then input into the loss function, by which they are compared to the corresponding ground truth labels (i.e., the high-quality target data 153). During the backward pass, the model computes the gradient of the loss function with respect to the current parameters, after which the parameters are updated by taking a step of size of a predefined size in the direction of minimized loss (e.g., in accelerated methods, such that the Nesterov momentum method and various adaptive methods, the step size can be selected to more quickly converge to optimize the loss function).

The optimization method by which the backprojection is performed can use one or more of gradient descent, batch gradient descent, stochastic gradient descent, and mini-batch stochastic gradient descent. The forward and backwards passes can be performed incrementally through the respective layers of the network.

In the forward pass, the execution starts by feeding the inputs through the first layer, thus creating the output activations for the subsequent layer. This process is repeated until the loss function at the last layer is reached. During the backward pass, the last layer computes the gradients with respect to its own learnable parameters (if any) and also with respect to its own input, which serves as the upstream derivatives for the previous layer. This process is repeated until the input layer is reached.

Returning to Figure 5, step 317 of training process 160 determines a change in the error as a function of the change in the network can be calculated (e.g., an error gradient), and this change in the error can be used to select a direction and step size for a subsequent change to the weights/coefficients of the DL network 170.

Calculating the gradient of the error in this manner is consistent with certain implementations of a gradient descent optimization method. In certain other implementations, this step can be omitted and/or substituted with another step in accordance with another optimization algorithm (e.g., a non-gradient descent optimization algorithm like simulated annealing or a genetic algorithm), as would be understood by one of ordinary skill in the art.

In step 317 of training process 160, a new set of coefficients are determined for the DL network 170. For example, the weights/coefficients can be updated using the changed calculated in step 317, as in a gradient descent optimization method or an over-relaxation acceleration method.

In step 318 of training process 160, a new error value is calculated using the updated weights/coefficients of the DL network 170.

In step 319, predefined stopping criteria are used to determine whether the training of the network is complete. For example, the predefined stopping criteria can evaluate whether the new error and/or the total number of iterations performed exceed predefined values.

For example, the stopping criteria can be satisfied if either the new error falls below a predefined threshold or if a maximum number of iterations is reached. When the stopping criteria is not satisfied the training process performed in training process 160 will continue back to the start of the iterative loop by returning and repeating step 317 using the new weights and coefficients (the iterative loop includes steps 317, 318, and 319). When the stopping criteria are satisfied the training process performed in training process 160 is completed.

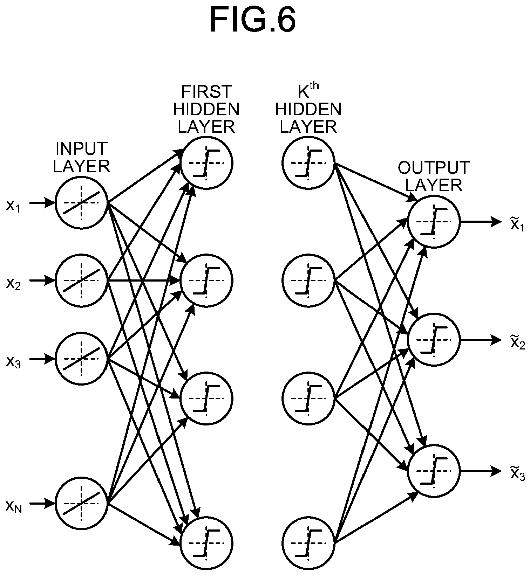

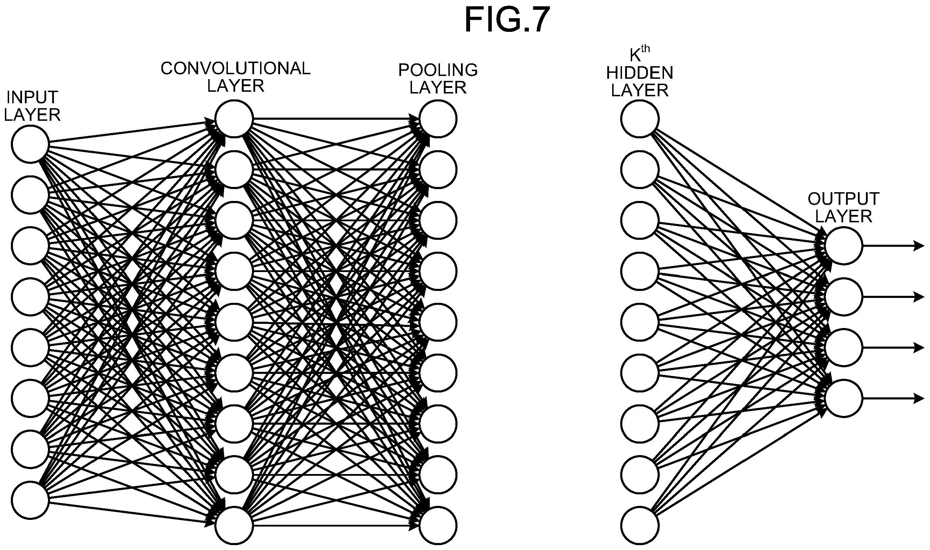

Figures 6 and 7 show two examples of the inter-connections between layers in the DL network 170. The DL network 170 can include fully connected, convolutional, and the pooling layer, all of which are explained below. In certain preferred implementations of the DL network 170, convolutional layers are placed close to the input layer, whereas fully connected layers, which perform the high-level reasoning, are place further down the architecture towards the loss function. Pooling layers can be inserted after convolutions and proved a reduction lowering the spatial extent of the filters, and thus the amount of learnable parameters.

Activation functions are also incorporated into various layers to introduce nonlinearity and enable the network to learn complex predictive relationships. The activation function can be a saturating activation functions (e.g., a sigmoid or hyperbolic tangent activation function) or rectified activation function (e.g., the Rectified Linear Unit (ReLU) applied in the first and second examples discussed above). The layers of the DL network 170 can also incorporate batch normalization, as also exemplified in the first and second examples discussed above.

Figure 6 shows an example of a general artificial neural network (ANN) having $N$ inputs, $K$ hidden layers, and three outputs. Each layer is made up of nodes (also called neurons), and each node performs a weighted sum of the inputs and compares the result of the weighted sum to a threshold to generate an output. ANNs make up a class of functions for which the members of the class are obtained by varying thresholds, connection weights, or specifics of the architecture such as the number of nodes and/or their connectivity. The nodes in an ANN can be referred to as neurons (or as neuronal nodes), and the neurons can have inter-connections between the different layers of the ANN system.

The synapses (i.e., the connections between neurons) store values called "weights" (also interchangeably referred to as "coefficients" or "weighting coefficients") that manipulate the data in the calculations. The outputs of the ANN depend on three types of parameters: (i) the interconnection pattern between the different layers of neurons, (ii) the learning process for updating the weights of the interconnections, and (iii) the activation function that converts a neuron's weighted input to its output activation.

Mathematically, a neuron's network function $m(x)$ is defined as a composition of other functions $n_i(x)$, which can further be defined as a composition of other functions. This can be conveniently represented as a network structure, with arrows depicting the dependencies between variables, as shown in Figure 6. For example, the ANN can use a nonlinear weighted sum, wherein $m(x) = K(\Sigma_i w_i n_i(x))$, where $K$ (commonly referred to as the activation function https://en.wikipedia.org/wiki/Artificial_neural_network - cite_note-30) is some predefined function, such as the hyperbolic tangent.

In Figure 6 (and similarly in Figure 7), the neurons (i.e., nodes) are depicted by circles around a threshold function. For the non-limiting example shown in Figure 6, the inputs are depicted as circles around a linear function, and the arrows indicate directed connections between neurons. In certain implementations, the DL network 170 is a feed-forward network.

Figure 7 shows a non-limiting example in which the DL network 170 is a convolutional neural network (CNN). CNNs are type of ANN that has beneficial properties for image processing, and, therefore, have specially relevancy for the applications of image denoising. CNNs use feed-forward ANNs in which the connectivity pattern between neurons can represent convolutions in image processing.

For example, CNNs can be used for image-processing optimization by using multiple layers of small neuron collections which process portions of the input image, called receptive fields. The outputs of these collections can then tiled so that they overlap, to obtain a better representation of the original image. This processing pattern can be repeated over multiple layers having alternating convolution and pooling layers.

Following after a convolutional layer, a CNN can include local and/or global pooling layers, which combine the outputs of neuron clusters in the convolution layers. Additionally, in certain implementations, the CNN can also include various combinations of convolutional and fully connected layers, with pointwise nonlinearity applied at the end of or after each layer.

In the embodiment described above, the case in which training of the DL network 170 that is the first trained model is performed by using projection data having a large focal spot size as an input and projection data having a small focal spot size as an output is explained. However, embodiments are not limited to this situation.

As an example, training of the DL network 170 that is the first train model may be further performed by using data acquired by using a detector array having detection elements of a first size as an input and a detector array having detection elements of a second size that is smaller than the first size as an output. In such a case, the DL network 170 that is the first train model consists of a first DL network that performs training regarding the focal spot size and a second DL network that performs training regarding the detection element size. Training is independently performed for the first DL network and for the second DL network, and the trained model is generated.

In the execution stage of the trained model, that is process 100, the raw data 251 is, for example, data acquired by using a first focal spot size that is a large focal spot size and a detector array having detection elements of a first size that is a large detection element size. The generation circuitry 520 outputs, by inputting the raw data 251 to the DL network 170, data equivalent to data acquired by using a second focal spot size that is smaller than the first focal spot size and a detector array having detection elements of a second size that is smaller than the first size. As an example, the generation circuitry 520 inputs raw data 251 to the first DL network, inputs its output result to the second DL network to acquire the output result, thereby acquiring data equivalent to data acquired by using the second focal spot size that is smaller than the first focal spot size and a detector array having detection elements of a second size that is smaller than the first size.

Alternatively, training regarding the focal spot size and training regarding the detection element size may be performed concurrently.

For example, training of the DL network 170 that is first train model may be performed by using data acquired by using a first focal spot size and a detector array having detection elements of a first size as an input and using data acquired by using a second focal spot size that is smaller than the first focal spot size and a detector array having detection elements of a second size that is smaller than the first size.

Alternatively, the combination of data used for the training may be otherwise. For example, training of the DL network that is first train model may be performed by using data acquired by using a first focal spot size and a detector array having detection elements of a second size as an input and data acquired by using a second focal spot size and a detector array having detection elements of a first size as an output.

In the example described above, embodiments regarding X-ray systems are described. However, an image processing apparatus having an equivalent function to the generation circuitry 520, the reconstruction circuitry 514, storage device 512, display 516, input device 515 and the like of Figure 8, may constitute an independent image processing apparatus. Such image processing comprises generation circuitry having an equivalent function to the generation circuitry 520, which inputs, to a first trained model that is trained by using first projection data as an input and second projection data as an output, third projection data to generate a fourth projection data, the first projection data being acquired using an X-ray source having a first focal spot size, the second projection data being acquired using an X-ray source having a second focal spot size that is smaller than the first focal spot size, and the third projection data being acquired using an X-ray source having a third focal spot size that is larger than the second focal spot size. Further, a program executed in a computer may cause the computer to execute a step of: inputting, to a first trained model that is trained by using first projection data as an input and second projection data as an output, third projection data to generate a fourth projection data, the first projection data being acquired using an X-ray source having a first focal spot size, the second projection data being acquired using an X-ray source having a second focal spot size that is smaller than the first focal spot size, and the third projection data being acquired using an X-ray source having a third focal spot size that is larger than the second focal spot size.

In addition to the embodiments described above, the following embodiments are possible.

The first trained model may be trained using a first subtraction data between the first projection data and the second projection data as an output.

For example, the first trained model may be a residual network which is trained using first subtraction data between the first projection data and the second projection data and output second subtraction data by inputting the third projection data. The fourth projection data may be generated based on the second subtraction data and the third projection data. For example, the fourth projection data may be generated by subtracting the second subtraction data from the third projection data.

The first trained model may be a trained model on which training is performed by using data acquired using only a detector array having detection elements of a first size as an input and (1) using data acquired using only a detector array having detection elements of a second size (2) using both a detector array having detection elements of the first size and a detector array having detection elements of a second size or (3) using data acquired using only a detector array having detection elements of the first size and a detector array having detection elements of a second size as an output, the second size being smaller than the first size.

The first trained model may be a trained model on which training is performed by using data acquired using a detector array having detection elements of a first size and a detector array having detection elements of a second size as an input, and (1) using data acquired using a detector array having detection elements of the second size or (2) using data acquired using only a detector array having detection elements of the second size as an output, the second size being smaller than the first size.

The second size may be $1/n^2$ of the first size, wherein the n is an integer of 2 or more. For example, the second size may be 1/4 or 1/9 of the first size.

The first focal spot size may be equal to the third focal spot size or may be different from the third focal spot size.

The first trained model may be trained further using projection data being acquired using an X-ray source having a different focal spot size from the first focal spot size. The different focal spot size may be equal or similar to the third focal spot size.

The first focal spot size, the second focal spot size, and the third focal spot size may be determined by accepting a user instruction, or based on information such as protocol information or examination region information.

For example, The X-ray system may select a focal spot size (the first focal spot size, the second focal spot size, or the third focal spot size ) from among the plurality of focal spot sizes according to a user instruction, scan protocol information, or examination region information.

Further, when the X-ray system selects the third focal spot size, the circuitry may select the first trained model from among a plurality of trained models and apply acquired projection data with the selected focal spot size to the first trained model. In other words, trainings may be used using different trained models and one of the trained models may be selected according to the input of the third focal spot size.

Further, when the X-ray system selects a different focal spot size from the third focal spot size, the circuitry may select a different trained model from the first trained model from among the plurality of trained models and apply acquired projection data with the selected focal spot size to the different trained model. In other words, the circuitry may interpolate or extrapolate the results of different trained models to obtain data of various focal spot sizes.

Each of the second projection data and the fourth projection data may be replaced by CT image data. In other word, the circuitry may input, to a first trained model that is trained by using first projection data as an input and first CT image data as an output, second projection data to generate a second CT image data, the first projection data being acquired using an X-ray source having a first focal spot size, the first CT image data being acquired using an X-ray source having a second focal spot size that is smaller than the first focal spot size, and the second projection data being acquired using an X-ray source having a third focal spot size that is larger than the second focal spot size.

Each of the first projection data and the third projection data may be replaced by CT image data. In other word, the circuitry may input, to a first trained model that is trained by using first CT image data as an input and first projection data as an output, second CT image data to generate a second projection data, the first CT image data being acquired using an X-ray source having a first focal spot size, the first projection data being acquired using an X-ray source having a second focal spot size that is smaller than the first focal spot size, and the second CT image data being acquired using an X-ray source having a third focal spot size that is larger than the second focal spot size.

Each of the first projection data, the second projection data, the third projection data, and the fourth projection data may be replaced by CT image data. In other word, the circuitry may input, to a first trained model that is trained by using first CT image data as an input and second CT image data or first subtraction data between the first CT image data and the second CT image data as an output, third CT image data to generate a fourth CT image data, the first CT image data being acquired using an X-ray source having a first focal spot size, the second CT image data being acquired using an X-ray source having a second focal spot size that is smaller than the first focal spot size, and the third CT image data being acquired using an X-ray source having a third focal spot size that is larger than the second focal spot size.

According to at least one embodiment described above, it is possible to improve image quality.

While certain implementations have been described, these implementations have been presented by way of example only, and are not intended to limit the teachings of this disclosure. Indeed, the novel methods, apparatuses and systems described herein may be embodied in a variety of other forms; furthermore, various omissions, substitutions and changes in the form of the methods, apparatuses and systems described herein may be made without departing from the scope of the invention as defined by the appended claims.

**Claims**

1. A system comprising:

   circuitry configured to

   input, to a first trained model third projection data to generate fourth projection data,
   receive the third projection data, said third projection data obtained from a computed tomography, CT, scan,
   and reconstruct a first image based on the fourth projection data,
   wherein the first trained model has been trained using first projection data as an input and second projection data or first subtraction data between the first projection data and the second projection data as an output,

   the first projection data being acquired using an X-ray source having a first focal spot size,
   the second projection data being acquired using an X-ray source having a second focal spot size that is smaller than the first focal spot size, and the third projection data being acquired using an X-ray source having a third focal spot size that is larger than the second focal spot size, **characterised in that**
   the first trained model is a trained model on which further training is performed by using data acquired using a detector array having detection elements of a first size as an input and using data acquired using detection elements of a second size as an output, the second size being smaller than the first size.

2. The system according to claim 1, wherein
   the X-ray system generates a second image by denoising the first image, said denoising being performed by inputting the first image into a second trained model.

3. The system according to claim 1, wherein

   the first projection data and the second projection data are sinograms and
   the first image is a CT image.

4. The system according to at least one of claims 1 to 3, wherein

   the second projection data is data acquired by averaging data detected by a plurality of detection elements of a detector array, and
   a signal to noise ratio of the second projection data is larger than a signal to noise ration of the first projection data.

5. The system according to at least one of claims 1 to 4, wherein

   the second projection data is acquired by using a smaller point spread function than a point spread function of the first projection data, and
   a resolution of the fourth projection data is higher than a resolution of the third projection data.

6. The system according to at least one of claims 1 to 5, wherein

   training of the first trained model is performed using a residual network and
   the circuitry generates the fourth projection data by subtracting an output of the residual network from the third projection data.

7. The system according to claim 1, wherein

   the first projection data and the second projection data are sinograms and
   the trained model is trained such that weighting coefficients of a neural network is iteratively adjusted such that a loss function is minimized, the loss function being determined based on a disagreement between output sinogram and the second projection data, the output sinogram being output based on the first projection data.

8. The system according to claim 7, wherein
   the loss function is at least one of a peak to noise ratio, a structural similarity index and an $l_p$-norm of a difference between the output sinogram and the second projection data.

**9.** The system according to at least one of claims 1 to 8, wherein
the third projection data is fluoroscopy data.

**10.** The system according to at least one of claims 1 to 8, wherein

the third projection data is X-ray CT projection data, and further comprising
a rotatable gantry, wherein the gantry comprises:

an X-ray tube configured on the gantry to rotate around an object, a size of a focal spot being controlled by an area of an electron beam on an anode and an angle of the anode with respect to a direction of an X-ray beam comprising X-rays emitted from the anode; and
a detector array including the plurality of detectors, the detector array being arranged on the gantry diametrically opposed to the X-ray tube and configured to rotate together with the X-ray tube, the detector array being further configured to receive the X-rays to generate the X-ray CT projection data.

**11.** A program that causes a computer to execute the steps of

receiving third projection data, said third projection data obtained from a computed tomography, CT, scan,
inputting, to a first trained model the third projection data to generate fourth projection data
reconstructing a first image based on the fourth projection data,
wherein the first trained model has been trained using first projection data as an input and second projection data or first subtraction data between the second projection data and the first projection data as an output, the first projection data being acquired using an X-ray source having a first focal spot size, the second projection data being acquired using an X-ray source having a second focal spot size that is smaller than the first focal spot size, and the third projection data being acquired using an X-ray source having a third focal spot size that is larger than the second focal spot size, **characterised in that**
the first trained model is a trained model on which further training is performed by using data acquired using a detector array having detection elements of a first size as an input and using data acquired using detection elements of a second size as an output, the second size being smaller than the first size.

**Patentansprüche**

**1.** System, umfassend:
eine Schaltung, die dafür konfiguriert ist:

dritte Projektionsdaten in ein erstes trainiertes Modell einzugeben, um vierte Projektionsdaten zu erzeugen,
die dritten Projektionsdaten zu empfangen, wobei die dritten Projektionsdaten aus einem Computertomographie-, CT-, Scan erhalten werden, und
auf der Grundlage der vierten Projektionsdaten ein erstes Bild zu rekonstruieren,
wobei das erste trainierte Modell unter Verwendung erster Projektionsdaten als eine Eingabe und zweiter Projektionsdaten oder erster Subtraktionsdaten zwischen den ersten Projektionsdaten und den zweiten Projektionsdaten als eine Ausgabe trainiert worden ist,
wobei die ersten Projektionsdaten unter Verwendung einer Röntgenquelle mit einer ersten Brennfleckgröße erlangt werden, die zweiten Projektionsdaten unter Verwendung einer Röntgenquelle mit einer zweiten Brennfleckgröße, die kleiner als die erste Brennfleckgröße ist, erlangt werden und die dritten Projektionsdaten unter Verwendung einer Röntgenquelle mit einer dritten Brennfleckgröße, die größer als die zweite Brennfleckgröße ist, erlangt werden, **dadurch gekennzeichnet, dass**
das erste trainierte Modell ein trainiertes Modell ist, an dem weiteres Training durchgeführt wird, indem Daten, die unter Verwendung einer Detektoranordnung mit Detektionselementen einer ersten Größe ermittelt wurden, als eine Eingabe verwendet werden und Daten, die unter Verwendung von Detektionselementen einer zweiten Größe ermittelt wurden, als Ausgabe verwendet werden, wobei die zweite Größe kleiner als die erste Größe ist.

**2.** System nach Anspruch 1, wobei
das Röntgensystem ein zweites Bild durch Entrauschen des ersten Bildes erzeugt, wobei das Entrauschen durch Eingeben des ersten Bildes in ein zweites trainiertes Modell durchgeführt wird.

**3.** System nach Anspruch 1, wobei

die ersten Projektionsdaten und die zweiten Projektionsdaten Sinogramme sind und das erste Bild ein CT-Bild ist.

4. System nach mindestens einem der Ansprüche 1 bis 3, wobei

die zweiten Projektionsdaten Daten sind, die durch Mittelwertbildung von Daten erlangt werden, die durch eine Vielzahl von Detektionselementen einer Detektoranordnung ermittelt wurden, und ein Signal-Rausch-Verhältnis der zweiten Proj ektionsdaten größer ist als ein Signal-Rausch-Verhältnis der ersten Projektionsdaten.

5. System nach mindestens einem der Ansprüche 1 bis 4, wobei

die zweiten Projektionsdaten durch Verwendung einer kleineren Punktspreizfunktion als eine Punktspreizfunktion der ersten Projektionsdaten erfasst werden, und eine Auflösung der vierten Projektionsdaten höher ist als eine Auflösung der dritten Projektionsdaten.

6. System nach mindestens einem der Ansprüche 1 bis 5, wobei

das Training des ersten trainierten Modells unter Verwendung eines Residuen-Netzes durchgeführt wird, und die Schaltung die vierten Projektionsdaten durch Subtrahieren einer Ausgabe des Residuen-Netzes von den dritten Projektionsdaten erzeugt.

7. System nach Anspruch 1, wobei

die ersten Projektionsdaten und die zweiten Projektionsdaten Sinogramme sind, und das trainierte Modell derart trainiert wird, dass Gewichtungskoeffizienten eines neuronalen Netzes iterativ angepasst werden, sodass eine Verlustfunktion minimiert wird, wobei die Verlustfunktion auf der Grundlage einer Nichtübereinstimmung zwischen dem ausgegebenen Sinogramm und den zweiten Projektionsdaten bestimmt wird, wobei das ausgegebene Sinogramm auf der Grundlage der ersten Projektionsdaten ausgegeben wird.

8. System nach Anspruch 7, wobei die Verlustfunktion mindestens eines von einem Spitzenwert-Rausch-Verhältnis, einem Index der strukturellen Ähnlichkeit und einer $l_p$-Norm einer Differenz zwischen dem ausgegebenen Sinogramm und den zweiten Projektionsdaten ist.

9. System nach mindestens einem der Ansprüche 1 bis 8, wobei die dritten Projektionsdaten Fluoroskopiedaten sind.

10. System nach mindestens einem der Ansprüche 1 bis 8, wobei

die dritten Projektionsdaten Röntgen-CT-Projektionsdaten sind, und ferner umfassend eine drehbare Gantry, wobei die Gantry Folgendes umfasst:

eine Röntgenröhre, die an der Gantry konfiguriert ist, um sich um ein Objekt zu drehen, wobei eine Größe eines Brennflecks durch einen Flächeninhalt eines Elektronenstrahls auf einer Anode und einen Winkel der Anode in Bezug auf eine Richtung eines Röntgenstrahls, der von der Anode emittierte Röntgenstrahlen umfasst, gesteuert wird, und eine Detektoranordnung, welche die Vielzahl von Detektoren einschließt, wobei die Detektoranordnung an der Gantry diametral gegenüber der Röntgenröhre angeordnet und dafür konfiguriert ist, sich zusammen mit der Röntgenröhre zu drehen, wobei die Detektoranordnung ferner dafür konfiguriert ist, die Röntgenstrahlen zu empfangen, um die Röntgen-CT-Projektionsdaten zu erzeugen.

11. Programm, das einen Computer veranlasst, die folgenden Schritte auszuführen:

Empfangen von dritten Projektionsdaten, wobei die dritten Projektionsdaten aus einem Computertomographie-, CT-, Scan erhalten werden, Eingeben der dritten Projektionsdaten in ein erstes trainiertes Modell, um vierte Projektionsdaten zu erzeugen, Rekonstruieren eines ersten Bildes auf der Grundlage der vierten Projektionsdaten, wobei das erste trainierte Modell unter Verwendung erster Projektionsdaten als eine Eingabe und zweiter Projektionsdaten oder erster Subtraktionsdaten zwischen den zweiten Projektionsdaten und den ersten Projektionsdaten als eine Ausgabe trainiert worden ist, wobei die ersten Projektionsdaten unter Verwendung einer Röntgenquelle mit einer ersten Brennfleckgröße erlangt werden, die zweiten Projektionsdaten unter Verwendung einer Röntgenquelle mit einer zweiten Brennfleckgröße, die kleiner als die erste Brennfleckgröße ist, erlangt werden und die dritten Projektionsdaten unter Verwendung einer Röntgenquelle mit einer dritten Brennfleckgröße, die größer als die zweite Brennfleckgröße ist, erlangt werden, **dadurch gekennzeichnet, dass**: das erste trainierte Modell ein trainiertes Modell ist, an dem weiteres Training durchgeführt wird, indem Daten, die unter Verwendung einer Detektoranordnung mit Detektionselementen einer ersten Größe ermittelt wurden, als eine Eingabe verwendet werden und Daten, die unter Verwendung von Detektionselementen einer zweiten Größe ermittelt wurden, als Ausgabe verwendet werden, wobei die zweite Größe kleiner als die erste Größe ist.

## Revendications

1. Système comprenant :

   un circuit configuré pour
   entrer, sur un premier modèle entraîné, des troisièmes données de projection pour générer des quatrièmes données de projection,
   recevoir les troisièmes données de projection, lesdites troisièmes données de projection étant obtenues à partir d'une tomodensitométrie, CT, et
   reconstruire une première image sur la base des quatrièmes données de projection,
   dans lequel le premier modèle entraîné a été entraîné en utilisant des premières données de projection en tant qu'entrée et des deuxièmes données de projection ou des premières données de soustraction entre les premières données de projection et les deuxièmes données de projection en tant que sortie,
   les premières données de projection étant acquises en utilisant une source de rayons X présentant une première dimension de tache focale, les deuxièmes données de projection étant acquises en utilisant une source de rayons X présentant une deuxième dimension de tache focale qui est plus petite que la première dimension de tache focale et les troisièmes données de projection étant acquises en utilisant une source de rayons X présentant une troisième dimension de tache focale qui est plus grande que la deuxième dimension de tache focale, **caractérisé en ce que**
   le premier modèle entraîné est un modèle entraîné sur lequel un entraînement ultérieur est réalisé en utilisant des données acquises en utilisant un réseau de détecteurs comportant des éléments de détection d'une première dimension en tant qu'entrée et en utilisant des données acquises en utilisant des éléments de détection d'une seconde dimension en tant que sortie, la seconde dimension étant plus petite que la première dimension.

2. Système selon la revendication 1, dans lequel
   le système rayons X génère une seconde image en débruitant la première image, ledit débruitage étant effectué en entrant la première image à l'intérieur d'un second modèle entraîné.

3. Système selon la revendication 1, dans lequel
   les premières données de projection et les deuxièmes données de projection sont des sinogrammes et la première image est une image CT.

4. Système selon au moins l'une des revendications 1 à 3, dans lequel

   les deuxièmes données de projection sont des données acquises en calculant la moyenne de données détectées par une pluralité d'éléments de détection d'un réseau de détecteurs, et
   un rapport signal sur bruit des deuxièmes données de projection est plus grand qu'un rapport signal sur bruit des premières données de projection.

5. Système selon au moins l'une des revendications 1 à 4, dans lequel

   les deuxièmes données de projection sont acquises en utilisant une fonction d'étalement du point d'une dimension plus petite qu'une fonction d'étalement du point des premières données de projection, et
   une résolution des quatrièmes données de projection est plus élevée qu'une résolution des troisièmes données de projection.

**6.** Système selon au moins l'une des revendications 1 à 5, dans lequel

l'entraînement du premier modèle entraîné est effectué en utilisant un réseau résiduel et
le circuit génère les quatrièmes données de projection en soustrayant une sortie du réseau résiduel vis-à-vis des troisièmes données de projection.

**7.** Système selon la revendication 1, dans lequel

les premières données de projection et les deuxièmes données de projection sont des sinogrammes et
le modèle entraîné est entraîné de telle sorte que des coefficients de pondération d'un réseau neuronal soient réglés par itérations de telle sorte qu'une fonction de perte soit minimisée, la fonction de perte étant déterminée sur la base d'une divergence entre un sinogramme de sortie et les deuxièmes données de projection, le sinogramme de sortie étant émis en sortie sur la base des premières données de projection.

**8.** Système selon la revendication 7, dans lequel
la fonction de perte est au moins l'un parmi un rapport crête sur bruit, un indice de similarité structurelle et une norme $l_p$ d'une différence entre le sinogramme de sortie et les deuxièmes données de projection.

**9.** Système selon au moins l'une des revendications 1 à 8, dans lequel
les troisièmes données de projection sont des données de fluoroscopie.

**10.** Système selon au moins l'une des revendications 1 à 8, dans lequel

les troisièmes données de projection sont des données de projection CT rayons X, et comprenant en outre un portique rotatif, dans lequel le portique comprend :

un tube rayons X configuré sur le portique pour tourner autour d'un objet, une dimension d'une tache focale étant contrôlée au moyen d'une aire d'un faisceau électronique sur une anode et d'un angle de l'anode par rapport à une direction d'un faisceau de rayons X comprenant des rayons X émis depuis l'anode ; et
un réseau de détecteurs incluant la pluralité de détecteurs, le réseau de détecteurs étant agencé sur le portique en opposition diamétrale par rapport au tube rayons X et étant configuré pour tourner en association avec le tube rayons X, le réseau de détecteurs étant en outre configuré pour recevoir les rayons X pour générer les données de projection CT rayons X.

**11.** Programme qui amène un ordinateur à exécuter les étapes suivantes :

la réception de troisièmes données de projection, lesdites troisièmes données de projection étant obtenues à partir d'une tomodensitométrie, CT,
l'entrée, sur un premier modèle entraîné, des troisièmes données de projection pour générer des quatrièmes données de projection,
la reconstruction d'une première image sur la base des quatrièmes données de projection,
dans lequel le premier modèle entraîné a été entraîné en utilisant des premières données de projection en tant qu'entrée et des deuxièmes données de projection ou des premières données de soustraction entre les deuxièmes données de projection et les premières données de projection en tant que sortie, les premières données de projection étant acquises en utilisant une source de rayons X présentant une première dimension de tache focale, les deuxièmes données de projection étant acquises en utilisant une source de rayons X présentant une deuxième dimension de tache focale qui est plus petite que la première dimension de tache focale et les troisièmes données de projection étant acquises en utilisant une source de rayons X présentant une troisième dimension de tache focale qui est plus grande que la deuxième dimension de tache focale, **caractérisé en ce que** le premier modèle entraîné est un modèle entraîné sur lequel un entraînement ultérieur est réalisé en utilisant des données acquises en utilisant un réseau de détecteurs comportant des éléments de détection d'une première dimension en tant qu'entrée et en utilisant des données acquises en utilisant des éléments de détection d'une seconde dimension en tant que sortie, la seconde dimension étant plus petite que la première dimension.

# FIG.1A

# FIG.1B

# FIG.2

# FIG.3A

ELECTRON BEAM
CROSS-SECTION

ANODE

X-RAY

ELECTRON BEAM

PROJECTED FOCAL SPOT

# FIG.3B

ELECTRON BEAM
CROSS-SECTION

ANODE

X-RAY

ELECTRON BEAM

PROJECTED FOCAL SPOT

# FIG.3C

ELECTRON BEAM
CROSS-SECTION

ANODE

X-RAY

ELECTRON BEAM

PROJECTED FOCAL SPOT

# FIG.4

<u>10</u>

# FIG.5

160

316

INITIALIZE THE NETWORK. CALCULATE AN ERROR BETWEEN THE OPTIMIZED DATA AND THE NETWORK PROCESSED NOISY/ARTIFACT DATA.

317

CALCULATE CHANGE IN ERROR AS A FUNCTION OF CHANGE IN THE NETWORK COEFFICIENTS. UPDATE THE NETWORK.

318

CALCULATE NEW ERROR BETWEEN THE OPTIMIZED DATA AND THE NEW NETWORK PROCESSED NOISY/ARTIFACT DATA.

319

NO

STOPPING CRITERIA REACHED?

YES

# FIG.6

# FIG.7

INPUT
LAYER

CONVOLUTIONAL
LAYER

POOLING
LAYER

Kth
HIDDEN
LAYER

OUTPUT
LAYER

EP 3 764 325 B1

# FIG.8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JAN KUNTZ et al.** Focal spot deconvolution using convolutional neural networks. *Proc. of SPIE,* March 2019, vol. 10948